(19) Europäisches Patentamt
European Patent Office
Office européen des brevets

(11) **EP 3 068 493 B1**

(12) **FASCICULE DE BREVET EUROPEEN**

(45) Date de publication et mention de la délivrance du brevet:
**27.05.2020 Bulletin 2020/22**

(21) Numéro de dépôt: **14814927.1**

(22) Date de dépôt: **13.11.2014**

(51) Int Cl.:
*A61Q 3/02* (2006.01)          *A61K 8/88* (2006.01)
*A61K 8/02* (2006.01)

(86) Numéro de dépôt international:
**PCT/FR2014/052888**

(87) Numéro de publication internationale:
**WO 2015/071596 (21.05.2015 Gazette 2015/20)**

(54) **UTILISATION DE POUDRE DE POLYAMIDE DANS UNE COMPOSITION COSMÉTIQUE POUR LES ONGLES**

VERWENDUNG VON POLYAMIDPULVER IN EINER KOSMETISCHEN ZUSAMMENSETZUNG FÜR NÄGEL

USE OF POLYAMIDE POWDER IN A COSMETIC COMPOSITION FOR NAILS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.11.2013 FR 1361079**

(43) Date de publication de la demande:
**21.09.2016 Bulletin 2016/38**

(73) Titulaire: **Arkema France 92700 Colombes (FR)**

(72) Inventeurs:
- **CHEMINET, Helena F-27300 Bernay (FR)**
- **LAPORTE, Stéfanie F-27300 Bernay (FR)**

(74) Mandataire: **Schaefer, Anne-Sophie et al Arkema France Département Propriété Industrielle 420, rue d'Estienne d'Orves 92705 Colombes Cedex (FR)**

(56) Documents cités:
EP-A1- 1 000 607          EP-A1- 2 420 222
WO-A1-00/27347          FR-A1- 2 903 595
JP-A- 2001 233 730          US-A- 5 747 018

- **"Orgasol- cosmetic Ingredients", INTERNET CITATION, 1 mars 2008 (2008-03-01), pages 1-8, XP002568536, Extrait de l'Internet: URL:http://www.arkema.com/pdf/EN/products/ technical_polymers/orgasol/brochure_Orgaso l_cosmetics_2008.pdf [extrait le 2010-02-12]**
- **GALLOUEDEC F D C: "Optimization of Ultrafine Microporous Powders to Obtain Low-Gloss UV Curable Coatings", RADTECH REPORT, NORTHBROOK, IL, US, 1 septembre 1995 (1995-09-01), pages 18-24, XP008124131, ISSN: 1056-0793**

**Description**

La présente invention concerne le domaine des compositions destinées à réparer et/ou à décorer des ongles, notamment les compositions cosmétiques pour les ongles, telles que les vernis à ongles

En plus d'exigences classiquement recherchées pour ces compositions pour ongles, telles que séchage rapide, tenue longue durée, résistance mécanique, résistance aux rayures, anti-choc, ou encore effet fortifiant de l'ongle avec une ou plusieurs vitamines par exemple, les formulateurs cherchent des moyens d'adapter facilement l'aspect mât/brillant des vernis, l'aspect doux ou rugueux, permettant d'obtenir ainsi des effets spéciaux, tels que des effets mats, rugueux, craquelés, marbrés, « peau de croco », etc, selon les dernières tendances de la mode. La décoration des ongles devient véritablement un art (« nail art ») qui nécessite des compositions de formulations toujours plus innovantes, applicables au pinceau, au stylo, au pochoir, ou bien selon d'autres méthodes, plus ou moins sophistiquées, notamment par réticulation sous UV.

La présente invention a donc pour but de fournir de telles compositions cosmétiques apportant de nouveaux touchers et/ou de nouveaux visuels sur l'ongle, c'est ce qu'on entend par « effets spéciaux » au sens de l'invention, tout en répondant aux autres exigences classiquement recherchées citées plus haut.

La présente invention a également pour but de fournir un procédé simple de fabrication de telles compositions cosmétiques à effets spéciaux sur l'ongle, qui présente le moins d'étapes possibles, et qui n'altère pas les autres propriétés cosmétiques exigées classiquement pour ces compositions pour ongles.

On a maintenant montré qu'il était possible de fabriquer de telles compositions cosmétiques pour ongles, permettant d'obtenir des revêtements à effets spéciaux sur l'ongle, grâce à l'addition de poudres de polyamide particulières, en jouant notamment sur la granulométrie, la teneur et le type de poudre de polyamide utilisée.

Dans l'art antérieur, les poudres polymères sont utilisées pour améliorer différentes propriétés des cosmétiques. Le document D1 FR2903595 concerne des compositions cosmétiques de maquillage et/ou de soin des matières kératiniques comprenant une poudre de polymère thermoplastique, ainsi que l'utilisation de cette poudre pour améliorer les propriétés de compositions cosmétiques, telles que le toucher et/ou l'aspect, la résistance à l'abrasion et/ou l'adhérence. L'exemple 13 un vernis comprenant de 0,5% à 1% en poids de poudre PA de diamètre moyen de 10 $\mu$m. Le document D2 JP2001233730 exemple 4 décrit un vernis à ongles à effets luminescents, comprenant 10% en poids de poudre Orgasol 2002 EXTD de diamètre moyen d'environ 12 $\mu$m. Le document D3 US5747018 concerne l'utilisation de poudres de diamètre maximal 50 $\mu$m, par exemple en polyamide, comme agents épaississants des vernis à ongles, ou pour améliorer l'application uniforme, la vitesse de séchage et l'adhésion aux ongles naturels dans ces vernis. Le document D4 EP1000607 décrit un procédé de maquillage dans lequel on applique une couche de particules solides insolubles sur une première couche de composition filmogène.

Les poudres de polyamide sont parfois évoquées dans l'art antérieur comme charge pour modifier la rhéologie des compositions liquides de vernis à ongles mais l'utilisation de poudres de polyamide particulières pour obtenir des effets spéciaux sur l'ongle vernis n'y est pas abordée, en particulier l'aspect rugueux.

La présente invention a donc pour objet l'utilisation de particules de poudre de polyamide dans une composition cosmétique pour les ongles pour améliorer l'aspect glissant et/ou l'aspect rugueux d'une couche de ladite composition sur l'ongle, dans laquelle :

-   le diamètre moyen en volume des particules est compris dans la gamme de 80 à 120 $\mu$m,
-   la teneur en poudre de PA est comprise dans la gamme de de 2 à 10%, en poids sur le poids total de la composition.

La poudre est additionnée directement à la dite composition, et mélangée éventuellement, par exemple pas simple agitation.

Le diamètre moyen en volume ou mesure de la granulométrie des poudres dans la présente description, notamment dans les exemples et comparatifs, est réalisée à l'aide d'un granulomètre de marque Coulter LS230. Il permet d'obtenir la distribution granulométrique des poudres de laquelle on peut déterminer le diamètre moyen et la largeur de la distribution ou l'écart-type de la distribution. La distribution granulométrique des poudres selon l'invention est déterminée selon les techniques habituelles à l'aide d'un granulomètre Coulter LS230 de la société Beckman-Coulter. A partir de la distribution granulométrique, il est possible de déterminer le diamètre moyen en volume avec le mode de calcul logarithmique version 2.11a. du logiciel, ainsi que l'écart-type qui mesure le resserrement de la distribution ou la largeur de la distribution autour du diamètre moyen.

La composition ainsi additivée forme une base pour vernis, un vernis de maquillage pour les ongles, une composition de finition pour les ongles et/ou une composition de soin cosmétique pour les ongles, ladite composition selon l'invention étant pigmentée ou transparente.

Selon un mode de réalisation préféré de l'invention, lesdites particules de polyamide sont choisies parmi les particules de polyamide, de copolyamide, et leurs mélanges.

Par particules de polyamide (homopolyamide ou copolyamide) au sens de l'invention on entend les produits de condensation des lactames, des aminoacides et/ou des diacides avec les diamines et, en règle générale, tout polymère formé par des motifs reliés entre eux par des groupes amides. Les particules selon l'invention peuvent également être issues de la copolymérisation de lactame(s) avec une ou plusieurs lactone(s) conduisant à un copolyesteramide comme décrit dans le brevet EP1172396.

Le terme « monomère » dans la présente description des copolyamides doit être pris au sens d' « unité répétitive ». Le cas où une unité répétitive du polyamide est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser. Dans le cas où les particules selon l'invention comprenant au moins deux monomères différents, appelés «co-monomères», c'est à dire au moins un monomère et au moins un co-monomère (monomère différent du premier monomère), elles forment un copolymère tel qu'un copolyamide abrégé CoPA.

A titre d'exemple de lactames, on peut citer ceux ayant de 3 à 12 atomes de carbone sur le cycle principal et pouvant être substitués. On peut citer par exemple le $\beta,\beta$-diméthylpropriolactame, le $\alpha,\alpha$-diméthylpropriolactame, l'amylolactame, le caprolactame, le capryllactame, l'oenantholactame, le 2-pyrrolidone et le lauryllactame.

A titre d'exemple de diacide (ou acide dicarboxylique), on peut citer les acides ayant entre 4 et 18 atomes de carbone. On peut citer par exemple, l'acide adipique, l'acide sébacique, l'acide azélaïque, l'acide subérique, l'acide isophtalique, l'acide butanedioïque, l'acide 1,4 cyclohexyldicarboxylique, l'acide téréphtalique, le sel de sodium ou de lithium de l'acide sulphoisophtalique, les acides gras dimérisés(ces acides gras dimérisés ont une teneur en dimère d'au moins 98% et sont de préférence hydrogénés) et l'acide dodécanédioïque HOOC-(CH2)10-COOH.

A titre d'exemple de diamine, on peut citer les diamines aliphatiques ayant de 6 à 12 atomes, elle peut être arylique et/ou cyclique saturée. A titre d'exemples on peut citer l'hexaméthylènediamine, la pipérazine, la tetraméthylène diamine, l'octaméthylène diamine, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPDM), la bis(aminocyclohéxyl) méthane (BACM), la bis(3-méthyl-4 aminocyclohéxyl) méthane (BMACM), la méthaxylyènediamine, le bis-p aminocyclohexylméthane et la triméthylhexaméthylène diamine.

A titre d'exemple d'aminoacide, on peut citer les alpha-oméga aminoacides, tels que les acides aminocaproïque, amino-7-heptanoïque, amino-11-undécanoïque, n-heptyl-11-aminoundécanoïque et amino-12-dodécanoïque.

A titre d'exemple de lactone, on peut citer la caprolactone, la valérolactone et la butyrolactone.

Le ou les monomère(s) préférentiellement utilisé(s) dans l'invention est ou sont choisi(s) parmi les lactames tels que par exemple le lauryllactame, le caprolactame, l'oenantholactame, le capryllactame ou leurs mélanges. De préférence, on utilise le lauryllactame seul ou en mélange avec le caprolactame.

De préférence, les particules de polyamide selon l'invention comprennent, une teneur en pourcentage molaire de polyamide 12 comprise dans la gamme allant de 50% à 100%, de préférence de 80% à 100%.

De préférence, lesdites particules de poudre à base de polyamide sont obtenues au moins en partie par polymérisation anionique, par ensemencement avec une charge minérale ou organique, de lactame(s) et/ou de lactone(s) en solution et/ou en suspension dans un liquide organique. On pourra se référer par exemple aux procédés décrits dans les documents EP0192515 et FR2910900. Avantageusement, lesdites particules poreuses dans la présente invention sont sphéroïdales. Elles sont préférentiellement choisies parmi les poudres commercialisées sous la marque Orgasol®.

De manière alternative, les particules de poudre à base de polyamide sont obtenues au moins en partie par polymérisation hydrolytique, puis broyage en poudres, généralement de formes irrégulières, sans arête vive. De telles poudres sont préférentiellement choisies parmi les poudres commercialisées sous la marque Rilsan®.

Selon un mode de réalisation avantageux de la présente invention, le PA est choisi parmi le PA11, PA 12, PA10.10, les copolyamides comprenant au moins un des monomères suivants : 6, 11, 12, 10.10, 10.12, 10.36, 6.10, 6.12, 10.T, et leurs mélanges.

La présente invention a également pour objet une composition cosmétique pour les ongles, comprenant des particules de poudre de polyamide, caractérisée en ce que :

- Le diamètre moyen en volume des particules est compris dans la gamme de 1 à 120 $\mu$m,
- La teneur en poudre de PA est comprise dans la gamme de 0,1 à 15% en poids sur le poids total de la composition, et caractérisée en ce qu'elle forme un film résistant aux rayures après application sur l'ongle puis séchage.

Selon un mode de réalisation avantageux de la composition de la présente invention,

- le diamètre moyen en volume des particules est compris dans la gamme de 80 à 120 $\mu$m, de préférence de 100 à 120 $\mu$m,
- la teneur en poudre de PA est comprise dans la gamme de 2 à 10%, de préférence de 3 à 8%, en poids sur le poids total de la composition ;

et ladite composition est caractérisée en ce qu'elle forme un film rugueux après application sur l'ongle puis séchage.

De préférence dans ce cas le PA comprend une teneur en pourcentage molaire de polyamide 11, 10.10 ou 10.12 comprise dans la gamme allant de 50% à 100%, de préférence de 80% à 100%.

La présente invention a également pour objet une composition cosmétique telle que celles définies ci-dessus, caractérisée en ce qu'il s'agit d'une base pour vernis, un vernis de maquillage pour les ongles, une composition de finition pour les ongles et/ou une composition de soin cosmétique pour les ongles, ladite composition selon l'invention pouvant être pigmentée ou transparente.

La présente invention concerne aussi bien la préparation de base-coats (visant à lisser l'ongle), que de topcoats (visant la protection du vernis), ou de vernis (pigmentés ou transparents), que ces produits soient en phase aqueuse, phase solvant ou composés à 100% d'extraits secs, et que les films de vernis soient obtenus par simple évaporation de la phase solvant organique ou aqueuse d'une solution ou dispersion d'au moins un polymère filmogène ou par réticulation sous UV.

La composition selon l'invention renferme un ou plusieurs des principaux constituants suivants couramment utilisés dans les vernis à ongles : agents filmogènes, plastifiants, solvants organiques ou aqueux, diluants, pigments, nacres et/ou composés colorants, agents thixotropants (ou thyxotropes).

La composition peut comprendre en outre au moins un des composés suivants : des agents épaississants minéraux ou organiques, des agents anti-UV, des agents mouillants, des agents d'étalement, des agents hydratants, des vitamines et/ou des parfums.

Exemples

Les exemples ci-dessous illustrent la présente invention sans en limiter la portée. Dans les exemples, sauf indication contraire, tous les pourcentages et parties sont exprimés en masse.

La formulation de référence est indiquée dans le tableau 1 suivant. Les ingrédients sont ajoutés dans l'ordre indiqué par les lignes du tableau.

*Tableau 1*

|   | % | Ingrédients | Fonction | Fournisseur |
|---|---|---|---|---|
|   | 1.000 | Tixogel LG-M | agent thixo trope | Rockwood |
| a | 18.000 | Ethyl Acetate | solvant | Brenntag |
|   | 4.000 | Isopropanol | diluant | Brenntag |
|   |   |   |   |   |
|   | 13.000 | NC ECOCELL 70% ds IPA | nitrocellulose | Safic-Alcan |
| d | 35.000 | Butyl Acetate | solvent | Brenntag |
|   | 4.000 | Isopropanol | diluant | Brenntag |
|   |   | Ajout de (a) dans (d) |   |   |
|   |   |   |   |   |
| e | 12.000 | Synolac 9614 BA 70 | résine polyester | Arkema |
| f | 6.000 | Citrofol BII | plastifiant | Jungbunzlauer |
|   |   |   |   |   |
| g | 5.000 | Butyl Acetate | solvant | Brenntag |
|   |   |   |   |   |
| i | 2.000 | Colorona Bordeaux | pigment | Merck |

Dans les exemples, la formule de référence du tableau 1 est additivée par chacun des polyamides suivants (grades Orgasol commercialisés par Arkema). Dans chaque cas, la poudre est simplement ajoutée directement à la formule de référence ci-dessus, selon les teneurs, grades et granulométries indiqués ci-après.

Pour les poudres Orgasol® :

- 3% 2002 UD Nat Cos : PA12; 5 $\mu$m

- 3% 2002 EXD Nat Cos : PA12; 10 $\mu$m
- 1,5%, 3% ou 5% 2002 D Nat Cos : PA12 ; 20 $\mu$m
- 3% Caresse : copolyamide 6/12 ; 10 $\mu$m
- 3% 2002 ES4 Nat 3 : PA12 ; 40 $\mu$m
- 3% 2002 ES6 Nat 3 : PA12 ; 60 $\mu$m

Pour les poudres Rilsan® :

- 3% T Nat BHV Cos : PA11; 104-114 $\mu$m.

Les revêtements obtenus après application de la même quantité de vernis sur l'ongle, sèchent plus vite dans le cas des compositions de l'invention grâce à l'addition de poudre PA, que dans le cas de la composition de référence sans poudre PA.

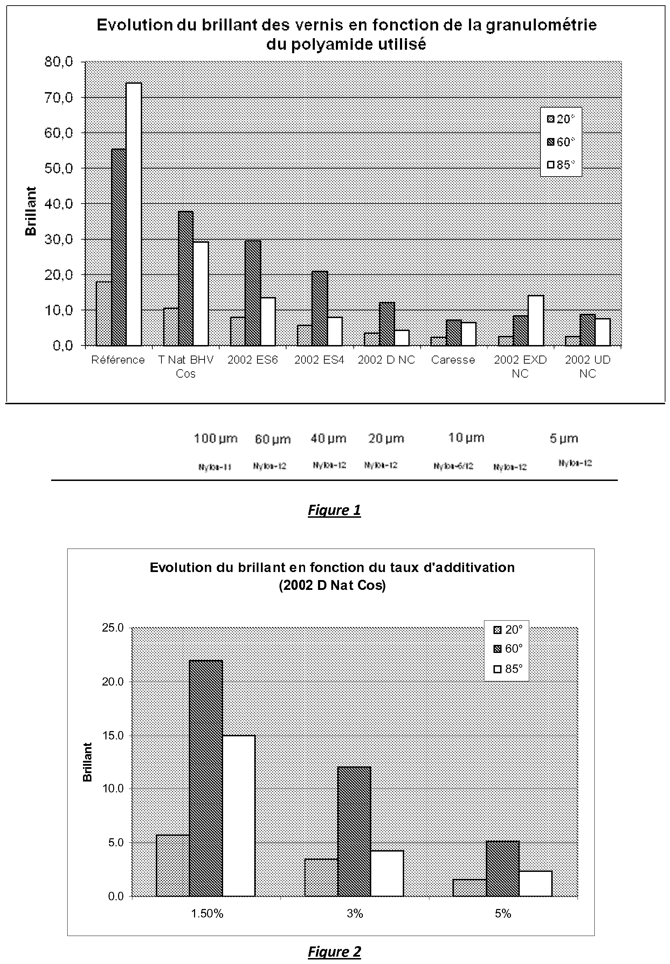

L'évolution du brillant du vernis respectivement en fonction de la granulométrie et en fonction du taux d'additivation de poudre de polyamide est représentée sur le graphe de la figure 1 et sur le graphe de la figure 2 respectivement.

On observe que l'aspect mât (contraire de brillant) augmente quand la granulométrie de la poudre de PA utilisée selon l'invention diminue (figure 1), et/ou quand la teneur en poudre de PA utilisée selon l'invention augmente (figure 2).

Dans la présente invention, l'aspect brillant (et donc au contraire l'aspect mât) est mesuré au moyen d'un réflectomètre, tel que le brillancemètre Dr LANGE REFO utilisé ici dans les exemples, pour 3 angles de mesures : 20°, 60° et 85°, déterminés par rapport à la normale d'incidence perpendiculaire au plan de l'éprouvette (plaque recouverte d'une couche de vernis sec de 50 $\mu$m d'épaisseur). La mesure du niveau de brillant correspond au pourcentage d'énergie lumineuse réfléchie à l'angle opposé du rayon lumineux émis, par rapport à l'énergie émise initialement. Le brillancemètre effectue automatiquement l'émission, la mesure et les calculs correspondants.

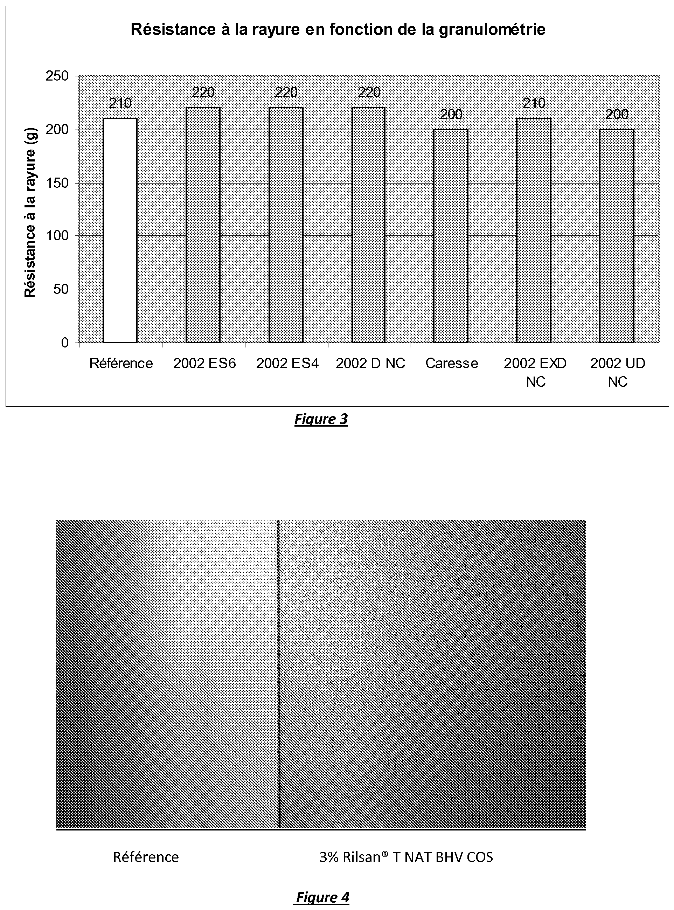

La résistance à la rayure du vernis en fonction de la granulométrie de la poudre est représentée sur le graphe de la figure 3. Cette figure montre que l'addition de poudre de PA dans la formule de vernis de référence ne dégrade pas la résistance à la rayure du revêtement obtenu après application et séchage de la composition sur l'ongle par rapport au revêtement obtenu avec la formule de référence. De plus, pour une même teneur en poudre PA additivée à la formule de référence, plus la granulométrie de la poudre augmente, plus la résistance à la rayure augmente.

La figure 4 représente l'aspect de rugosité (plaque de droite) du vernis obtenu par addition de 3% de poudre de polyamide 11 de diamètre moyen en volume 104-114 $\mu$m (Rilsan® T Nat BHV Cos) à la formulation de référence.

Le tableau 2 suivant montre les coefficients de friction ou glissement statique et dynamique mesurés sur des revêtements obtenus respectivement à partir de la composition de référence sans poudre de PA et à partir de la composition de référence additivée par 3% d'Orgasol® 2002 EXD Nat Cos selon l'invention.

Dans la présente invention de même que pour les exemples, le coefficient de friction est mesuré au moyen d'un dispositif Davenport qui déplace à vitesse constante (15 cm/min) un palet (200g) sur une couche de 50 $\mu$m de vernis sec sur une plaque en aluminium. La plaque en aluminium revêtue de vernis a été préalablement séchée et stockée à l'air libre (23°C, 50% humidité) pendant 7 jours.

Le coefficient de glissement statique (sans unité) correspond à la force nécessaire pour mettre le palet en mouvement (démarrage initial) :

$$\text{(Valeur lue (en g) x sensibilité (ici 2) ) / poids du palet (200g)}$$

Le coefficient de glissement dynamique (sans unité) correspond à la force nécessaire pour maintenir le déplacement continu du palet après le début du glissement :

$$\text{(Valeur lue (en g) x sensibilité (ici 2) ) / poids du palet (200g)}$$

On note une amélioration sensible du glissant en dynamique comme en statique, grâce à l'addition de poudre de PA selon l'invention.

*Tableau 2*

|  | Composition de référence | Composition de référence + 3% 2002 EXD NAT COS |
|---|---|---|
| Coefficient de friction statique | 0,46 | 0,41 |

(suite)

|  | Composition de référence | Composition de référence + 3% 2002 EXD NAT COS |
|---|---|---|
| Coefficient de friction dynamque | 0,36 | 0,27 |

## Revendications

**1.** Utilisation de particules de poudre de polyamide dans une composition cosmétique pour les ongles pour améliorer l'aspect brillant, et/ou l'aspect rugueux d'une couche de ladite composition sur l'ongle, dans laquelle :

- le diamètre moyen en volume des particules est compris dans la gamme de 80 à 120 $\mu$m,
- la teneur en poudre de PA est comprise dans la gamme de 2 à 10%, en poids sur le poids total de la composition,
- la poudre est additionnée directement à la dite composition,
- la composition ainsi additivée est une base pour vernis, un vernis de maquillage pour les ongles, une composition de finition pour les ongles et/ou une composition de soin cosmétique pour les ongles, ladite composition étant pigmentée ou transparente.

**2.** Utilisation selon la revendication 1, **caractérisée en ce que** le PA est choisi parmi le PA11, PA 12, PA10.10, les copolyamides comprenant au moins un des monomères suivants : 6, 11, 12, 10.10, 10.12, 10.36, 6.10, 6.12, 10.T, et leurs mélanges.

**3.** Utilisation selon la revendication 1 ou 2, dans laquelle :

- le diamètre moyen en volume des particules est compris dans la gamme de 100 à 120 $\mu$m,
- la teneur en poudre de PA est comprise dans la gamme de 3 à 8%, en poids sur le poids total de la composition ; **caractérisée en ce que** la composition additivée forme un film rugueux après application sur l'ongle puis séchage.

**4.** Utilisation selon la revendication 3, **caractérisée en ce que** dans la composition le PA comprend une teneur en pourcentage molaire de polyamide 11, 10.10 ou 10.12 comprise dans la gamme allant de 50% à 100%, de préférence de 80% à 100%.

**5.** Utilisation selon l'une quelconque des revendications 1 à 4, **caractérisée en ce que** la composition est une base pour vernis, un vernis de maquillage pour les ongles, une composition de finition pour les ongles et/ou une composition de soin cosmétique pour les ongles, ladite composition étant pigmentée ou transparente.

**6.** Utilisation selon la revendication 5, **caractérisée en ce que** la composition renferme en outre un ou plusieurs des constituants suivants couramment utilisés dans les vernis à ongles : agents filmogènes, plastifiants, solvants organiques ou aqueux, diluants, pigments, nacres et/ou composés colorants, agents thixotropants.

**7.** Utilisation selon la revendication 5 ou 6, **caractérisée en ce que** la composition renferme en outre au moins un des composés suivants : agents épaississants minéraux ou organiques, agents anti-UV, agents mouillants, agents d'étalement, agents hydratants, vitamines et/ou parfums.

## Patentansprüche

**1.** Verwendung von Polyamidpulverteilchen in einer kosmetischen Zusammensetzung für die Nägel zur Verbesserung des glänzenden Aussehens und/oder des rauen Aussehens einer Schicht der Zusammensetzung auf dem Nagel, wobei:

- der volumenmittlere Durchmesser der Teilchen im Bereich von 80 bis 120 $\mu$m liegt,
- der Gehalt an PA-Pulver im Bereich von 2 bis 10 Gew.-%, bezogen auf das Gesamtgewicht der Zusammensetzung, liegt,
- das Pulver direkt zu der Zusammensetzung gegeben wird,

- die so additivierte Zusammensetzung eine Lackgrundlage, ein Make-Up-Lack für die Nägel, eine Finishzusammensetzung für die Nägel und/oder eine kosmetische Pflegezusammensetzung für die Nägel ist, wobei die Zusammensetzung pigmentiert oder transparent ist.

2. Verwendung nach Anspruch 1, **dadurch gekennzeichnet, dass** das PA aus PA11, PA12, PA10.10, Copolyamiden, die mindestens eines der folgenden Monomere umfassen: 6, 11, 12, 10.10, 10.12, 10.36, 6.10, 6.12, 10.T, und Mischungen davon ausgewählt ist.

3. Verwendung nach Anspruch 1 oder 2, wobei:

- der volumenmittlere Durchmesser der Teilchen im Bereich von 100 bis 120 $\mu$m liegt,
- der Gehalt an PA-Pulver im Bereich von 3 bis 8 Gew.-%, bezogen auf das Gesamtgewicht der Zusammensetzung, liegt;
**dadurch gekennzeichnet, dass** die additivierte Zusammensetzung nach Aufbringen auf den Nagel und anschließendem Trocknen einen rauen Film bildet.

4. Verwendung nach Anspruch 3, **dadurch gekennzeichnet, dass** das PA in der Zusammensetzung einen Molprozentgehalt an Polyamid 11, 10.10 oder 10.12 im Bereich von 50 % bis 100 %, vorzugsweise von 80 % bis 100 %, umfasst.

5. Verwendung nach einem der Ansprüche 1 bis 4, **dadurch gekennzeichnet, dass** die Zusammensetzung eine Lackgrundlage, ein Make-Up-Lack für die Nägel, eine Finishzusammensetzung für die Nägel und/oder eine kosmetische Pflegezusammensetzung für die Nägel ist, wobei die Zusammensetzung pigmentiert oder transparent ist.

6. Verwendung nach Anspruch 5, **dadurch gekennzeichnet, dass** die Zusammensetzung außerdem einen oder mehrere der folgenden Bestandteile, die gemeinhin in Nagellacken verwendet werden, enthält: Filmbildner, Weichmacher, organische oder wässrige Lösungsmittel, Verdünnungsmittel, Pigmente, Perlglanzmittel und/oder Farbmittel, Thixotropiermittel.

7. Verwendung nach Anspruch 5 oder 6, **dadurch gekennzeichnet, dass** die Zusammensetzung außerdem mindestens eine der folgenden Verbindungen enthält: anorganische oder organische Verdickungsmittel, Anti-UV-Mittel, Befeuchtungsmittel, Verteilungsmittel, Feuchtigkeitsmittel, Vitamine und/oder Duftstoffe.

**Claims**

1. Use of polyamide powder particles in a cosmetic composition for the nails for improving the glossy appearance and/or the rough appearance of a layer of said composition on the nail, wherein :

- the volume-average diameter of the particles is within the range from 80 to 120 $\mu$m,
- the content of PA powder is within the range from 2 to 10% by weight, with regard to the total weight of the composition,
- the powder is added directly to said composition,
- the composition thus additivated is a varnish base, a makeup varnish for the nails, a finishing composition for the nails and/or a cosmetic care composition for the nails, said composition being pigmented or transparent.

2. Use according to Claim 1, **characterized in that** the PA is chosen from PA11, PA12, PA10.10, the copolyamides comprising at least one of the following monomers: 6, 11, 12, 10.10, 10.12, 10.36, 6.10, 6.12 and 10.T, and their blends.

3. Use according to Claim 1 or 2, wherein:

- the volume-average diameter of the particles is within the range from 100 to 120 $\mu$m,
- the content of PA powder is within the range from 3 to 8% by weight, with regard to the total weight of the composition;
**characterized in that** the additivated composition forms a rough film after application to the nail and then drying.

4. Use according to Claim 3, **characterized in that**, in the composition, the PA comprises a content, as molar percentage, of polyamide 11, 10.10 or 10.12 within the range extending from 50% to 100%, preferably from 80% to 100%.

5. Use according to any one of Claims 1 to 4, **characterized in that** the composition is a varnish base, a makeup varnish for the nails, a finishing composition for the nails and/or a cosmetic care composition for the nails, said composition being pigmented or transparent.

6. Use according to Claim 5, **characterized in that** the composition additionally includes one or more of the following constituents commonly used in nail varnishes: film-forming agents, plasticizers, organic or aqueous solvents, diluents, pigments, pearlescent agents and/or colouring compounds, and thixotropic agents.

7. Use according to Claim 5 or 6, **characterized in that** the composition additionally includes at least one of the following compounds: inorganic or organic thickening agents, UV stabilizers, wetting agents, spreading agents, moisturizing agents, vitamins and/or fragrances.

**Figure 1**

**Figure 2**

**Résistance à la rayure en fonction de la granulométrie**

Figure 3

Référence                    3% Rilsan® T NAT BHV COS

Figure 4

## RÉFÉRENCES CITÉES DANS LA DESCRIPTION

**Documents brevets cités dans la description**

- FR 2903595
- JP 2001233730 B
- US 5747018 A
- EP 1000607 A
- EP 1172396 A
- EP 0192515 A
- FR 2910900